(19)

**Europäisches Patentamt**
**European Patent Office**
**Office européen des brevets**

(11) **EP 1 362 606 B1**

(12) **EUROPEAN PATENT SPECIFICATION**

(45) Date of publication and mention
of the grant of the patent:
**14.04.2010 Bulletin 2010/15**

(51) Int Cl.:
***A61M 5/142*** *(2006.01)* *A61M 5/168* *(2006.01)*

(21) Application number: **02700619.6**

(86) International application number:
**PCT/JP2002/001460**

(22) Date of filing: **20.02.2002**

(87) International publication number:
**WO 2002/066102 (29.08.2002 Gazette 2002/35)**

(54) **SYRINGE PUMP**

SPRITZENPUMPE

POMPE A SERINGUE

(84) Designated Contracting States:
**AT BE CH CY DE DK ES FI FR GB GR IE IT LI LU MC NL PT SE TR**

(30) Priority: **22.02.2001 JP 2001045990**

(43) Date of publication of application:
**19.11.2003 Bulletin 2003/47**

(73) Proprietor: **TERUMO KABUSHIKI KAISHA**
**Tokyo 151-0072 (JP)**

(72) Inventor: **TACHIBANA, Yasuharu**
**Fuji-shi,**
**Shizuoka 417-0801 (JP)**

(74) Representative: **Paget, Hugh Charles Edward et al**
**Mewburn Ellis LLP**
**33 Gutter Lane**
**London**
**EC2V 8AS (GB)**

(56) References cited:
**EP-A1- 0 916 353      EP-A2- 1 110 569**
**JP-A- 2000 350 782    US-A1- 5 242 408**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a syringe pump which delivers (administers) a liquid medicine to a patient on the basis of a predetermined liquid infusing (administration) pattern and can change a blockage pressure detection level according to a pusher position.

BACKGROUND ART

There have been proposed syringe pumps which inject a liquid medicine on the basis of a set infusing rate by selecting beforehand an occlusion pressure detection level from a plurality of regions (for example, Japanese Patent Registration No. 2785114). Furthermore, there have been proposed fluid infusion devices which raise an alarm before the completion of injection by detecting a pusher position (for example, the Japanese publication of examined patent application No. 62-44505, Japanese publication of examined patent application No. 1-17380, etc.). However, due to the pusher position detection accuracy of a syringe pump and variations in the syringe size, with pusher position detection means alone, it has been difficult to accurately detect the position in which the infusion (or administration) of a liquid medicine in the syringe is completed.

EP-A-1110569 describes a syringe pump for infusion of medicine in a syringe, which forms part of the state of the art under Article 54(3) EPC, and which has input means for a liquid infusion rate and also an input means for selection and changing of an occlusion pressure level. A drive motor is controlled to cause infusion by pushing a pusher of the syringe, in accordance with the set infusion rate.

Disclosure of the Invention

Therefore, the present invention was made in view of the above-described situation and has as its object the provision of a syringe pump which can change a occlusion pressure detection level (a threshold value) according to a detected pusher position.

To achieve the above-described object, the syringe pump of the invention is as set out in claim 1.

Incidentally, the present invention is not limited to the embodiments described below.

BRIEF DESCRIPTION OF DRAWINGS

Figure 1 is an external perspective view of a syringe pump 1 of the invention;
Figure 2 (A) is an external perspective view showing how a syringe S is set in a syringe pump 1, and Figure 2 (B) is an external perspective view to explain the operation of a clamp 5 which detachably holds a syringe S in a syringe pump 1 in an immobile state;
Figure 3 is a front view showing a rough construction of a slider mechanism which drives a slider assembly 50;
Figure 4 is a plan view of an operating panel part 2f;
Figure 5 is a flow chart showing blockage detection; and
Figure 6 is a block diagram of a syringe pump of the invention.

BEST MODE FOR CARRYING OUT THE INVENTION

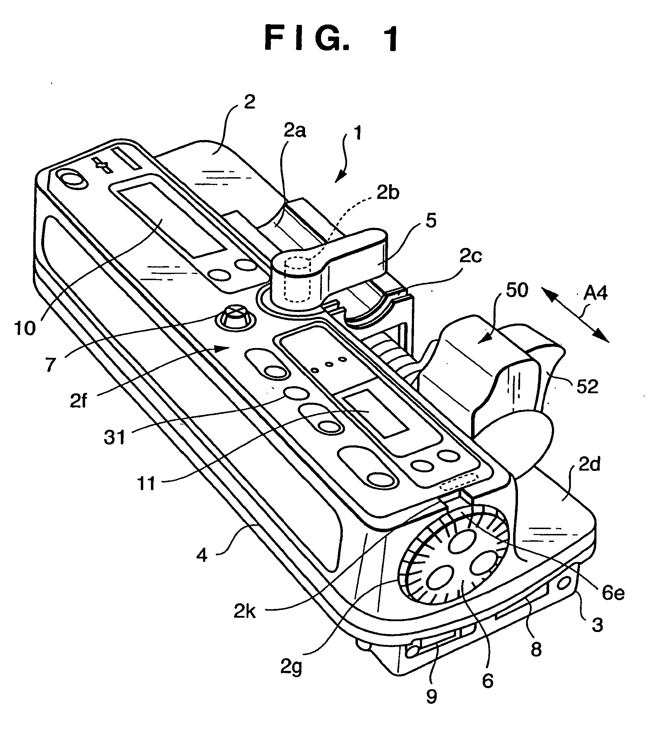

Embodiments of the invention will be described below by referring to the accompanying drawings. Figure 1 is an external perspective view of a syringe pump 1. In this figure, an operating panel part 2f is installed forward so that a setting dial 6 for setting flow rate etc. which is usually operated with the right hand can be seen.

In this figure, this syringe pump 1 is a small amount liquid continuously infusing pump aimed at nutritional support, blood transfusion and infusing (administering) liquid medicines, such as chemotherapy drugs and anesthetics in an ICU (intensive care unit), a CCU (coronary care unit) and an NICU (neonatal intensive care unit), and the operating panel part 2f for performing flow rate display etc. is provided in a manner almost concentrated on the top surface as shown in the figure to ensure good operability.

This operating panel 2f is basically covered with an embossed sheet cover and drip-proof design is performed to satisfy the drip-proof test of JIS 0920. For example, this operating panel enables carelessly spilled liquid medicines etc. to be easily wiped off and has high drip-proofness to prevent liquid medicines etc. from entering the interior. For this reason, an upper cover 2 and a lower cover 3 are integrally molded from a molding resin material having high chemical resistance and are secured by screws, with a rubber seal 4 made of silicone elastomer, for example, interposed at the mutual connection surfaces of each of the covers 2, 3, thereby preventing foreign substance such as a liquid from entering the interior.

Furthermore, in order to attach importance to high accuracy of liquid infusing and an improvement in operability, the realization of precise infusion action (operation) control by microcomputer control is made possible. At the same time, by causing an action indicator 7, which is provided so as to protrude upward in a position readily seen from the outside, to light up or blink red or greenly in multicolor or perform display as a revolving light, it is ensured that the action status or alarm status can be monitored from a distance, whereby a carefully thought-out measure is taken for safety. Moreover, a buzzer is built in and various alarm functions are provided for safety.

Furthermore, because the syringe pump is compact and lightweight, it is easy to carry and is designed so as to be convenient for being used even in a case where multiple units are used at a time. In addition, by turning the setting dial 6 on the right-hand surface of the apparatus, the numerical setting of fluid infusion flow rate (ml/h), fluid infusion volume (ml), etc. corresponding to the rotation speed and rotation angle can be performed in a short time. On the other hand, by causing set numerical values to be displayed in a display part 11 of a display panel, the setting of numerical values of flow rate etc. can be easily changed by one action by operating the setting dial 6.

Moreover, this syringe pump has a shape which can permit multiple use (simultaneous use of multiple units), is easy to use, and has design which permits buildup. The size is small, for example, 110 mm in height, 322 mm in width and 115 mm in depth. The weight is about 1.8 kg. The power source is of three systems, i.e., a commercial power source, a built-in battery and 12V DC.

The charge time of the built-in battery is 15 H (hours). In order to facilitate replacement from the outside, the built-in battery is covered with a cover at the bottom of the lower cover 3 and detachably provided by being connector-connected. Furthermore, the replacement life is not less than 3 years and charge control is tricle charge. In addition, the prevention of excessive discharge and charge is realized by taking measures for cell breakage detection and cell breakage safety of the charge battery. A heat resistant nickel-cadmium (Ni-Cd) battery is used and a new battery can work for not less than 180 minutes until an alarm is raised and for not less than 210 minutes until a shutdown.

A display part 10 for the injection of a medicine per body weight in $\mu$g/kg/min, mg/kg/h, etc., the display part 11 for flow rate, predetermined amount and integrated amount, etc., are provided on the operating panel 2f. On the other hand, a concavity 6e is formed so that the setting dial 6 can be easily removed for cleaning, and the setting dial is so constructed that it can be removed by being moved outward, with the tiptoe put into this portion and rotated.

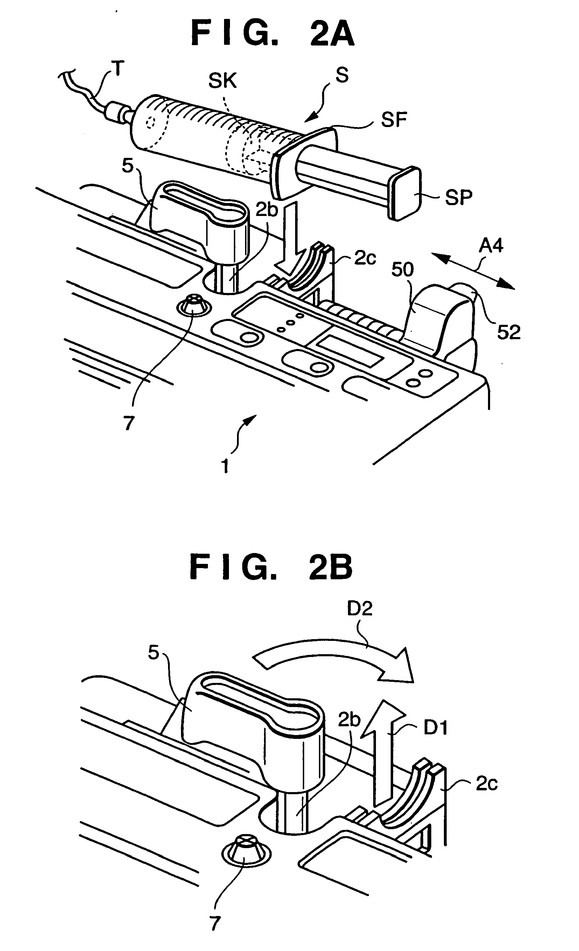

Next, in Figure 2(A), in mounting the syringe S to the syringe pump 1, a clamp 5 and a slider assembly 50 are moved beforehand to the positions shown in the figure, a flange SF of the main body in which a liquid medicine is housed is moved in the direction of the arrow and set in a slit 2c, a pusher SP is set in the slider assembly 50, and after that, the clamp 5 is set in such a condition that the clamp 5 pushed the main body of the syringe S, whereby the mounting is completed. In order to set the syringe S in this manner, in the upper cover 2 there are integrally formed a syringe stage 2a and the slit part 2c having a shape which permits the setting of the flange part SF of the syringe main body.

In order to ensure that various syringes S which are made by different manufacturers, or which have different outside dimensions and total lengths and different capacities, or which have a pusher SP in different positions can be handled at this time of mounting, the syringe pump is so constructed as to arbitrarily change the pushing position of the clamp 5 and the position of the slider assembly 50. For this purpose, as shown in Figure 2, the clamp 5 is fixed onto a freely rotatable shaft body 2b and constructed so as to bring the main body of the syringe S into a pressed condition by the action of a built-in spring (not shown) by being rotated in the direction of the arrow D2 after being manually drawn up in the direction of the arrow D1.

In order to move the slider assembly 50 which holds the pusher SP in an arbitrary position, the slider assembly 50 can be brought into a free state by pushing a clutch lever 52. Therefore, the pusher SP is set by manually moving the slider assembly 50. By starting the syringe pump after setting the syringe in this manner, the liquid medicine in the syringe S can be infused (delivered) via a tube T.

It is obligatory to provide the function of monitoring the pressure state during this infusion process. When occlusion occurs in the interior of the tube T etc. for some unexpected reason, such as a bend, a red light-emitting diode built in the above-described action indicator 7 rotates and blinks in order make this occlusion state known to a nurse and other persons.

Again, with reference to Figure 1, the slider assembly 50 driven in the direction of the arrow A4 in this figure reciprocally moves on a concavity 2d of the upper cover 2 and is fixed by being connected to a slider feed mechanism at the end portions of a pipe shaft and an inner clutch shaft, which will be described later. Therefore, by manually operating the clutch lever 52 of the slider assembly 50, the pusher SP of the syringe can be easily attached or detached.

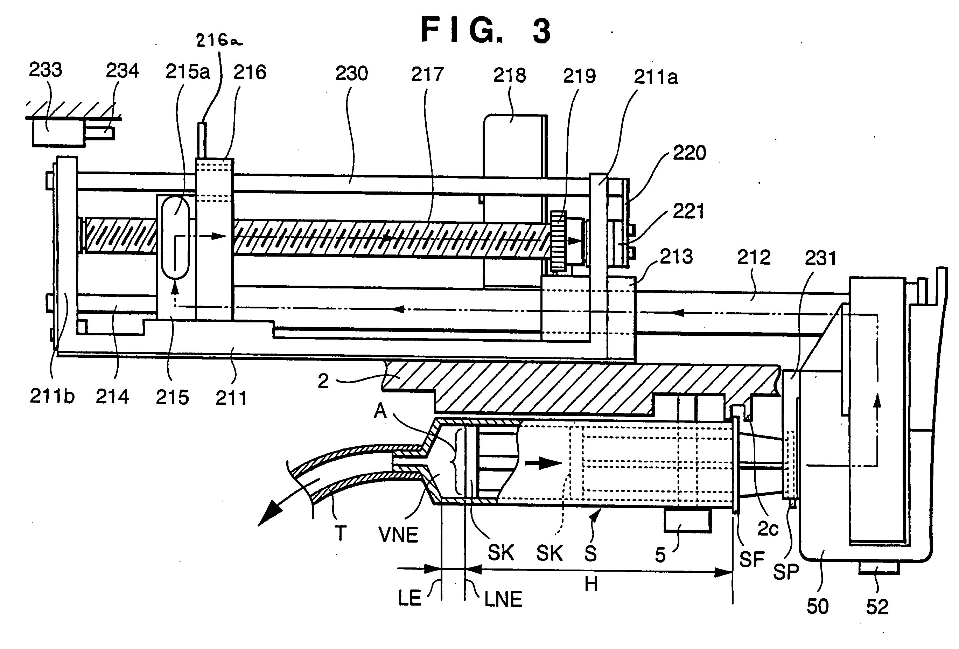

Next, Figure 3 is a plan view of a slider feed mechanism of the slider assembly 50. In this figure, mechanism parts which have already been described are given like reference characters and their descriptions are omitted. The slider assembly 50 uses as its mounting base a base 211 which is fixed to the case 2 in which the slit 2c is formed in a concave shape to receive a pressure that is a reaction force generated in the flange SF of the syringe S. And in left and right wall surface portions 211b, 211a of this base 211, an inner shaft 214 etc. are rotatably supported.

Around this inner shaft 214 there is provided a pipe shaft 212, at one end thereof is fixed the above-described slider assembly 50 and at the other end thereof are fixed a half nut holder 215 provided with a half nut 215a and a block 216 in such a manner that the half nut holder 215 is released from a state of meshing engagement with a tooth portion of a lead screw 217 by being rotated by the pressing operation of the above-described clutch lever 52. And the middle portion of this pipe shaft 212 is rotatably supported in a freely slidable manner by a sliding bearing 213 indicated with broken lines in the figure so that the slider assembly 50 is driven in one direction with the driving of the lead screw 217, whereby the pusher SP is moved to infuse (deliver) a liquid medicine through the tube T.

The left end of the shaft of this lead screw 217 is supported by a radial bearing which is not shown in the figure. Furthermore, in the vicinity of the right end of the lead screw 217, there is a gear 219 which is in meshing engagement with a gear fixed to an output shaft of a motor 218. The lead screw 217 passes through a hole drilled through a right wall surface portion 211a and the shaft of the lead screw 217 is freely rotatably supported by a bearing which is fixed to a spring plate 220, to which a pressure sensor 221 comprising a strain gauge which is the occlusion pressure detection means is fixed.

In the above-described configuration, it is possible to bring the half nut 216 into a state of meshing engagement with the lead screw 217 and a state of release from meshing engagement by operating the clutch lever 52, and, at the same time, occlusion detection is carried out by performing the detection of loads generated in the direction of the arrow by use of the pressure sensor 221 fixed to the end of the lead screw 217.

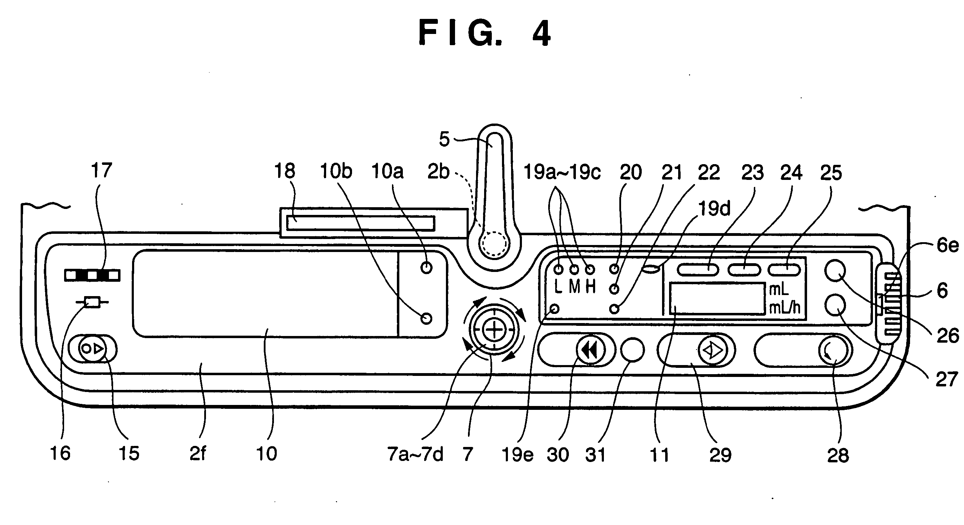

Next, Figure 4 is a plan view of an operating panel part 2f. In this figure, a power switch 15, an AC/DC lamp 16 and a battery lamp 17 are arranged in a centralized manner at the left end portion of the operating panel part 2f. Adjacent to these lamps is provided the display part of injection of medicines per body weight 10 which displays the injection of medicines per body weight. However, a model which is not provided this display part of injection of medicines per body weight 10 can be sufficiently used, such a model is also available. This display part of injection of medicines per body weight 10 is provided with a unit changeover switch (which serves also as a display on/off switch) 10a and an item changeover switch 10b.

On the other hand, on top of this display part 10 there is provided a syringe display lamp (a syringe display part) 18, which performs automatic measurement by use of syringe diameter detection means of the clamp 5 which detects the outside diameter (capacity) of the syringe after converting the vertical moving distance of the clamp into electrical signals upon setting of the syringe by use of the clamp 5 and displays the capacity (volume) of the set syringe in 10 cc (ml), 20 cc (ml), 30 cc (ml) and 50 cc (ml) and the syringe maker name.

Also, below the clamp 5 there is provided the action indicator 7 which is molded from a transparent acrylic resin etc. so as to have a shape protruding upward so that the action indicator can display the liquid feeding action state by electric light, for example, by the lighting up or blinking of built-in light-emitting diodes 7a to 7d which appropriately glow red and greenly or by the rotation in the direction indicated by the arrows in the figure so as to accomplish the scattering of light inside.

Adjacent to the clamp 5 are provided display lamps 19a, 19b, 19c, which display in a three-level switchover manner the set pressure detected by a occlusion detection mechanism provided in the syringe pump. Adjacent to these lamps, a remaining amount alarm lamp 20, a battery alarm lamp 22, etc. are arranged in a centralized manner. There are also provided a blockage pressure monitor display part 19d, which performs alarm display by lighting up by an LED etc. when the occlusion pressure becomes not less than a prescribed ratio (for example, not less than 50%) of a set value (mm Hg), and a pusher/clutch disengagement display part 19e, which displays the disengagement of the pusher and clutch.

Adjacent to these lamps, there is provided the 7-segment LED display part 11 which is provided with a flow rate lamp 23, a predetermined amount lamp 24 and an integrated amount lamp 25. In this display part 11, a maker name which is set and input by maker input means 40 or a maker name read by identification information reading means (not shown) is indicated by symbols or characters.

On the right side of this display part 11, there are provided a display changeover switch 26 and an integrating clearing switch 27. And below the display part 11 are provided a rapid feed switch 30, a start switch 29 and a stop/mute switch 28. There is also provided selection means (a selector switch) (not shown) which can select either or both of a remaining amount position alarm and a remaining time (finish time) alarm. Also, there is provided a manual switch 31 to maintain a medicine-in- blood concentration at a prescribed value when this concentration has reached the prescribed value. The medicine-in-blood concentration can be set at a prescribed value by pressing this manual switch 31 for a prescribed time (1 to 3 seconds).

To set the flange of the syringe in the slit 2c, the flange of the syringe is put into the slit 2c. After that, the main body of the syringe S is set on the syringe stage 2a and then clamping is performed by use of the clamp.

A small-diameter circular-arc groove portion is further integrally formed in the above-described slit 2c and syringe stage 2a so that a small-capacity, small-diameter syringe can be held in an immobile state. With the foregoing, the setting of the syringe is completed. By installing reading means which reads identification information (a bar code etc.) which is attached to the syringe and identifies the syringe maker and the syringe type including the syringe capacity at this time, it is possible to specify the syringe and/or the liquid medicine housed in the syringe.

Subsequently, the slider assembly 50 is moved by operating the clutch lever. When the clutch lever 52 of the slider assembly 50 shown in Figure 3 is pressed for this purpose, it becomes possible to manually move the slider assembly 50. Then, when the clutch lever 52 is released after the pusher SP of the syringe is brought into engagement with the slider assembly 50, a hook 231 in a pair of right and left portions comes to automatically hold the pusher SP of the syringes. That is, when the clutch lever 52 is released, the hook 231 comes to sandwich the pusher SP of the syringes. The foregoing is the basic setting action.

This slider feed mechanism is further provided with a blockage detection mechanism. A liquid medicine housed in the syringe can be injected by pushing the pusher of the syringe. At this time, due to the action of the resistance in the syringe and other fluid infusion passages, a force which acts in the reverse direction indicated by the arrow in Figure 3 is generated.

The force thus generated causes the deflection of the above-described pressure sensor 221 which uses a strain gauge etc. This mechanical deflection is converted into an electrical signal. When the value of this electrical signal reaches a set value (a threshold value) which is set for each maker and syringe size (syringe outside diameter) and stored in storage means (E$^2$PROM, RAM) 100, this condition is displayed on the action indicator 7 as an alarm to make the abnormal condition known to the user and an alarm sound (a buzzer) is generated (sounded) to alert the user.

The setting of a load thus generated from the resistance in the syringe and other fluid infusion passages is displayed in a three-level switchover manner by use of the three display lamps 19a, 19b, 19c provided in the display panel on the upper cover 2. The set load values are as follows:

(1) H (high): (800 $\pm$ 200 mm Hg)
(2) M (medium): (500 $\pm$ 100 mm Hg)
(3) L (small): (300 $\pm$ 100 mm Hg)

These set load values are presented in a table for each syringe maker (one or more table makers) in consideration of syringe outside diameter D(cm), syringe size (syringe capacity) (ml), syringe sectional area A (cm$^2$), sliding resistance of syringe Fs (kgf), etc. and stored in the storage means (memory) 100 as a threshold value P (kgf/cm$^2$).

Furthermore, a comparison is made between a converted value Fp of one selected threshold value P (kgf/cm$^2$) [= P (kgf/cm$^2$)·A (cm$^2$) + Fs (kgf)] on the basis for a syringe which is included in a table, set and specified and a value of pressure(load F (kgf)) which acts through the slider assembly 50 as indicated by the arrow in Figure 3 and detected by the above-described pressure sensor 221.

Preferably, this load F (kgf) is obtained by taking a predetermined number of samples, for example, 16 samples at predetermined sampling intervals, for example, every 0.05 second and obtaining a moving average thereby to remove noise components due to the backlash, etc. of the syringe and the drive part. The result thus obtained is segment displayed on the segment display part 11, and a warning (an alarm) to the effect that blockage at not less than a predetermined threshold value has occurred is generated and displayed by blinking etc.

On the other hand, a remaining amount detection/remaining amount position mechanism provides an important function for the case where the remaining amount becomes small during operation. When the pump continues its action and as a result the pusher SP of the syringe is moved and reaches an arbitrary position, the pusher 216a of the pump, which is arranged to move together with the pusher SP, comes into contact a lever 234 of a potentiometer 233 fixed to a frame.

When the movement is further continued from this contact condition, the value on the potentiometer 233 reaches a predetermined value which has been stored beforehand (a predicted alarm position which will be described later in detail). At this time, the threshold value of blockage detection is automatically set and changed by threshold value changing means 110. For example, when the above-described initial setting is H (high) [800 $\pm$ 200 mm Hg], this value becomes 300 $\pm$ 100 mm Hg; when the initial setting is M (medium)[500 $\pm$ 100 mm H], this value becomes (300 $\pm$ 100 mm Hg); and when the initial setting is L (small) [300 $\pm$ 100 mm Hg], this value becomes [300 $\pm$ 100 mm Hg], preferably, 30 to 100% of the initial setting. Furthermore, as an alarm to make known the fact that a planned injection is to be finished after a predetermined time (hour and minute), this is displayed in a remaining amount alarm lamp 21. And at the same time, either a remaining time (minute) or a remaining amount (ml) is displayed in a remaining time/remaining amount display part (not shown) or a remaining time (minute) and a remaining amount (ml) are alternately displayed and an alarm sound is generated and displayed.

When the liquid medicine infusion action of the pump is completed as described above, the slider assembly 50 is returned to the initial position by moving in the direction reverse to the feed direction. Incidentally, the lever 234 of the potentiometer 233 is so constructed as to return to its initial position by the force of a tension spring (not shown) connected to the lever.

Incidentally, if during the feed action of the pump the engagement state of the half nut 216 and lead screw 217 that constitute the clutch is cut when the user grips the clutch lever 52 by mistake or a similar phenomenon occurs for some action of load etc., the syringe pusher/clutch disengagement detection mechanism generates an alarm sound and causes the pusher/clutch disengagement display part 19e to blink, thereby making the abnormality known to the user.

Next, data on the syringe sectional area A (cm$^2$), push-finish position (push-finish completion position) LE (cm) and syringe outside diameter D (cm) is stored for each syringe S maker (one or more makers) and for each size. Also, data on syringe sectional area is stored and retained in an exchangeable memory (E$^2$PROM) within a CPU. Furthermore, there are provided the display part for the infusion (gamma infusion) of medicines per body weight and the display part for the infusion of a predetermined amount, alarms and a drive state. The alarm display is a three-dimensional display and a place lighted up by an LED rotates in a drive state.

The display of the remaining amount of a battery is performed by the battery lamp 17 as a three-level display. Conventionally, the detection of the remaining amount of a battery is detected on the basis of battery voltage. In this syringe pump, however, the remaining amount of a battery is detected at higher accuracy by the integration of current.

When the setting dial 6 is clockwise rotated, various set values appear in an increasing manner on the setting dial 6 and are displayed on the display part 11 shown in Figures 1 and 2. Similarly when setting dial 6 is anticlockwise rotated, set values are displayed on the display part 11 in a decreasing manner.

Concretely, when the display changeover switch 26 disposed on the display panel of Figure 4 is pressed, the flow rate lamp 23 and the prescribed amount lamp 24 light up sequentially and various kinds of setting can be performed.

In performing flow rate setting, the flow rate lamp 23 is brought into a lighting up state by pressing the display changeover switch 26 and after that, this setting is performed by rotating the setting dial 6. As a result, the display of set values is performed in the display part 11. The display range is 0.0 to 1200 ml/h (to 999.9: 0.1 ml/h steps) (1000 to: 1 ml/h steps). The minimum flow rate is 0.0 and it is possible to set the flow rate in a wide injection range. Furthermore, flow rate setting is ensured for various types of syringes of various makers. It is also ensured that when the flow rate set value is "0," the buzzer makes a one-shot sound and the action indicator lights up red instantaneously thereby to prevent a start. Also in a case where a set flow rate value exceeds a flow rate range which permits driving, the buzzer makes a one-shot sound and the action indicator lights up red instantaneously thereby to prevent a start.

An integrated amount is obtained by the integration of a pulse count value of an encoder (not shown) fixed to the output shaft of the motor 218 and also by the addition of a rapidly-fed amount which is rapidly fed after the pressing of the rapid feed switch 30.

An infused amount can be set by body weight in the range of 0.0 to 300.0 kg (in 0.1 kg steps), by an amount of medicine in the range of 0.0 to 999.9 mg (in 0.1 mg steps), by an amount of solution in the range of 0.0 to 999.9 ml (0.1 ml steps) and by a dose in the range of 0.00 to 99.99 ($\mu$g/kg/min or mg/kg/h) (0.01 steps).

A minimum flow rate is indicated from 0.0, and when body weight, an amount of medicine, an amount of solution and a dose are input, the flow rate is automatically calculated and displayed. The injection is started with the displayed flow rate. When this calculation result deviates from flow rates capable of being set (not less than 0.1 ml/h but not more than 1200 ml/h), the flow rate display of the display part 11 becomes a display other than numerals, i.e., [----], the buzzer makes a one-shot sound before and after this display change, and the action indicator lights up red instantaneously thereby to prevent the start of pump action.

On the other hand, the calculation result is displayed when the calculation result is within 1200 ml/h, whereas when the calculation result is not less than 300.1 ml/h and it is automatically detected that the syringe type is 10 to 30 cc, the buzzer makes a one-shot sound and the action indicator lights up red instantaneously thereby to prevent a start even when the start switch 29 is pressed.

Next, when the rapid feed switch 30 is pressed, the liquid infusion rate is increased and liquid infusing is performed at a rate of 1200 ml/h for a 50 cc syringe, at 500 ml/h for a 30 cc syringe, at 400 ml/h for a 20 cc syringe, and at 300 ml/h for a 10 cc syringe.

The display range for the setting of a predetermined amount is 0.0 to 999.9 ml (in 0.1 ml steps), and a minimum predetermined set value is 0.0. Action selection is made possible by use of an internal selector switch. In the case of a predetermined amount of 0.0, when the start switch 29 is pressed, the buzzer makes a one-shot sound, the action indicator lights up red instantaneously and, at the same time, the predetermined amount lamp 24 of the predetermined amount LED blinks thereby to prevent a start.

In the case of a predetermined amount s an integrated amount, the predetermined amount lamp 24 of the predetermined amount LED blinks and the buzzer makes a sound thereby to prevent a continuation. In the case of a predetermined amount ≤ an integrated amount during injection, the predetermined amount lamp 24 of the predetermined amount LED blinks, the buzzer makes a sound and the operation is automatically switched over to the KOR (keep open rate) run mode. Because at this time the flow rate is displayed as KOR (0.1 ml/h), the sound vanishes when the stop/mute switch 28 is pressed once. An alarm is given again during the continuation of this KOR operation, and the operation is stopped when the stop switch 28 is pressed again.

On the other hand, the initial display which appears when the power is made on for the syringe pump, is "0" ml except during the memory mode.

The order of priority of sounding is basically set so that priority decreases in the order of (a) occurrence of blockage, syringe disengagement and clutch disengagement, (b) a decrease in battery voltage (2 minutes, 4 minutes, 6 minutes, 8 minutes, 10 minutes, not less than 10 minutes), (c) a push-finish foretelling alarm (remaining amount foretelling alarm) and (d) a sound to announce a forgotten start. Furthermore, when multiple alarms have been given, a changeover is made to an alarm of higher order. Similarly, when multiple alarms have been given during the mute mode, it is ensured that an alarm of higher order is given and, at the same time, it is ensured that a key click sound and a one-shot sound are made in an interrupting manner while alarms are being intermittently given as with a sound to announce a forgotten start.

Again with reference to Figure 2, the action of the action indicator 7 is as follows. (a) During normal operation, the built-in light-emitting diodes 7a to 7d gradually blink greenly clockwise at four different speeds in response to set flow rates. (b) During the stop of operation, green lamps go out with the exception of red color lighting up during an alarm. (c) In the case of a sound to announce a forgotten start, all light-emitting diodes 7a to 7d blink red. (d) When communication from the outside is received, the lamps blink instantaneously.

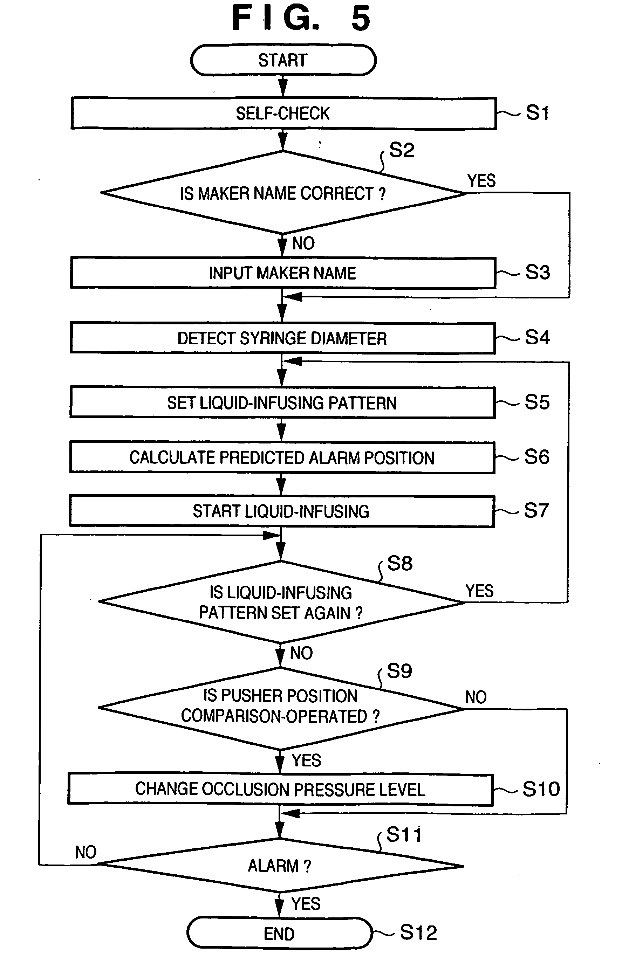

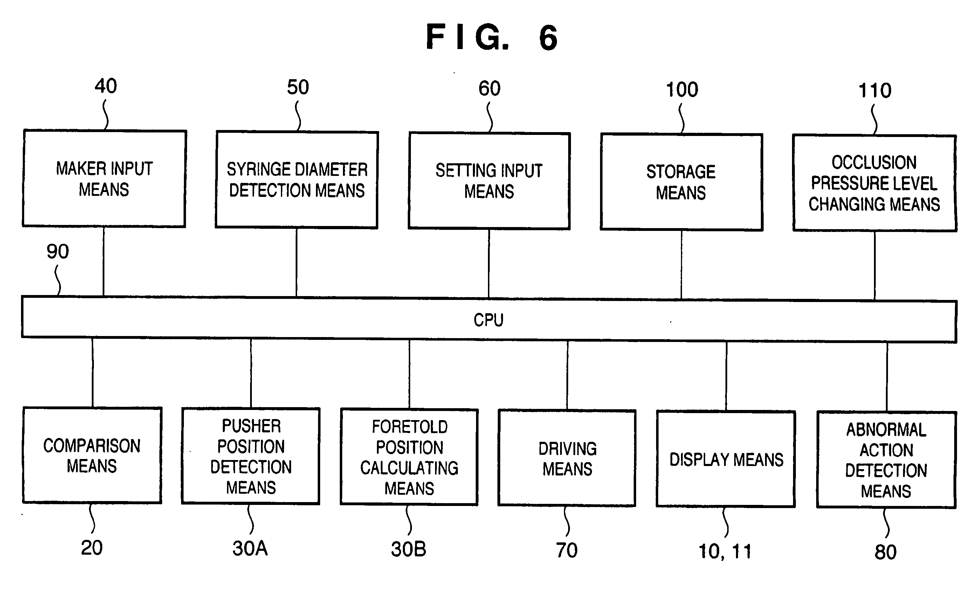

How to use the above-described syringe pump will be described below for the first embodiment by referring to the flow chart of Figure 5 and the block diagram of Figure 6.

To make preliminary arrangements, tools are first checked and it is ascertained that the syringe pump 1 and a pole clamp, an AC power cable, a fluid infusion stand, a syringe containing a liquid medicine and a lock needle, which are accessories, (all being not shown) are complete. After that, the pole clamp is fixed to the fluid infusion stand and the pole clamp is firmly fixed to the fluid infusion stand. For this purpose, an attaching screw of the pole clamp is put into a screw hole in the bottom of the syringe pump and fixed. Next, the AC power cable is connected to an AC inlet 8 on the right side surface of the main body and a plug is then connected to a 100 V AC receptacle provided with a ground terminal.

When the AC power is connected, the battery lamp 17 lights up and it is indicated that the built-in battery is being charged.

When the power is turned on by pressing the power switch 15 for about 1 second in order to put the power supply to work, all lamps blink three times, the buzzer sounds and self-check is automatically performed (Step S1). At this time, the AC/DC lamp 16 lights up and each of the display lamps of flow rate, predetermined amount and integrated amount 23, 24, 25 lights up. The syringe maker name which is set beforehand in the display part 11 of flow rate, predetermined amount and integrated amount is displayed by numerals for about 3 seconds. It is necessary to ascertain whether the set syringe corresponds to the syringe maker name (Step S2).

When the syringe maker name is inappropriate, a right maker name is input by use of the maker name input part (means) 40 (Step S3). The set and input maker name is displayed in the display part 11 by letters or symbols.

After a lapse of a predetermined time, the numerals of the syringe maker of the display part 11 of flow rate, predetermined amount and integrated amount disappear and the display part 11 indicates "0.0." The action indicator 7 is off at this time.

On the other hand, with all of the four built-in display lamps 18 of syringe type blinking, the display lamps indicate that the syringe S has not been mounted, thereby expediting the setting of the syringe. The setting of the syringe is started when all of the above-described indications have been ascertained. When the syringe filled with a liquid medicine is connected to the injection line (the tube T) in aseptic environment, the main body of the syringe is set on the stage 2a and engaged to this stage by use of the clamp 5.

Subsequently, as shown in Figure 3, the flange SF of the syringe S is put into the slit 2c in order to set the flange SF in the slit 2c. After that, the main body of the syringe S is set on the syringe stage 2a and the clamp 5 is then rotated in a predetermined direction. Then, the lock state is canceled and the clamping of the syringe is automatically performed and the syringe diameter (syringe capacity: 10 ml, 20 ml, 50 ml, etc.) is automatically detected by use of the syringe diameter detection means (detection part) 50 in a setting state (Step S4).

Subsequently, the slider assembly 50 is moved by pushing the clutch lever 52 of the slider assembly 50 thereby to release the clutch. At this time, when the clutch lever of the slider assembly 50 is pressed, the slider assembly 50 comes to be capable of being manually moved.

Then, the clutch lever 52 is released after the pusher SP of the syringe is caused to abut against the slider assembly 50, the pair of right and left hook 231 comes to automatically hold the pusher. That is, when the clutch lever is released, the pair of right and left hook comes to sandwich the pusher of the syringe.

When this syringe setting is finished, priming is performed. This priming must be performed by all means before the puncture of the patient. When the rapid feed switch 30 is pressed, the action indicator 7 rotates and performs indication and the pump action is started, with the result that the liquid medicine flows out of the tip of the lock needle. The liquid medicine is fed to the tip of the lock needle by continuously pushing this rapid feed switch 30. At this time the integrated amount lamp 25 blinks.

During rapid feed, the integrated amount lamp 25 blinks in the display part 11 of flow rate, predetermined amount and integrated amount and a priming amount is displayed in the display part 11 of flow rate, predetermined amount and integrated amount. This priming amount is added to an integrated amount in 0.1 ml steps. It is also possible to clear the integrated amount to "0" by pressing the integrating clearing switch 27.

This priming is important for ensuring that the working surface of the slider assembly 50 of the main body comes to engagement with the pusher SP of the syringe without a clearance by eliminating the clearance between the syringe and the main body and hence must be performed by all means.

When the above priming has been completed, a liquid infusing pattern (an infusion amount) is set by use of a setting and input means (the input part) 60 (Step S5). At this time, it is ascertained first that the flow rate lamp 23 is on. When this flow rate lamp 23 is not on, the flow rate lamp 23 is caused to light up by pressing the display changeover switch 26.

After that, a flow rate per hour is set by turning the setting dial 6 which constitutes part of the input part 60. At this time, in order to ensure safety by preventing a malfunction in the operation of the setting dial, a numeric value will not change during half a turn after the start of turning. When half a turn is exceeded, the buzzer sounds and the numeric value changes.

An increase or a decrease in this numeric value is such that the numeric value decreases when the setting dial 6 is turned forward, whereas the numeric value increases when the setting dial 6 is turned backward. A high-order numeral of a numeric value changes when the setting dial 6 is turned, with the stop/mute switch 28 kept pressed.

For the syringe type and maximum flow (infusion) rate, a maximum flow rate capable of being set is set depending on the syringe type and, for example, the maximum flow rate is 300 ml/h in the case of a 30 ml syringe. Therefore, when a numeric value larger than a maximum value is set and the star switch 29 is pressed, the set flow rate value blinks and infusion will not be started. Therefore, setting is performed again and completed.

The setting of an occlusion pressure detection level is performed by first pressing the stop/mute switch 28 and then pressing the display changeover switch 26. First, in changing the setting of an occlusion pressure detection level, "p***" appears in the display part 11 of flow rate, predetermined rate and integrated amount when the display changeover switch 26 is pressed while the stop/mute switch 28 is being pressed, with the result that the mode becomes the setting mode. With the stop/mute switch 28 kept depressed, the display changeover switch 26 is released and then pressed, the printed letters "L" (low), "M" (medium) and "H" (high) in the vicinity of the occlusion pressure set value lamps 19a, 19b, 19c light up sequentially. Therefore, a desired level of occlusion pressure is selected, set and input. Incidentally, the above *** are numerical values corresponding to the above L, M and H. In this manner, the stop/mute switch 28 and the display changeover switch 26 fulfill the function as the occlusion pressure selection means.

On the basis of data selected and input by the remaining amount/remaining time selector switch (selector means), a predicted alarm position LNE (cm) is calculated as follows in a control part (CPU) 90 (Step S6).

On the basis of the occlusion pressure level which has been selected and input and the syringe diameter (capacity) and syringe maker name, an occlusion pressure threshold value (an upper limit value and/or a lower limit value) which has been presented in a table beforehand is automatically selected and the infusing of the liquid medicine is started (Step S7).

A predetermined number of samples (for example, 16 samples) are continuously taken at prescribed cycles (for example, every 0.05 second), the moving average value of the samples is calculated, and a judgment is made as to whether the occlusion pressure exceeds the threshold value.

When the occlusion pressure exceeds the threshold value, an alarm (a warning) is given and the action indicator 7 lights up (blinks) red to make this known to the user. The user confirms this and performs resetting when there is no problem. When it is necessary to change the liquid infusing pattern during liquid infusion, the set value is changed and a new value is input (Step S8). Infusion is continued when the occlusion pressure does not exceed the threshold value.

<Setting at a remaining amount>

When setting at a remaining amount VNE (ml) of the syringe S to be set is performed by referring to Figure 3, the push-finish position LE (cm) is calculated on the basis of the above stored data presented in a table by using the following equation:

$$\texttt{Push-finish position LE (cm) = LNE (cm) + VNE (ml)/A (cm}^2\texttt{),}$$

where LNE (cm) is the alarm position and A (cm$^2$) is the syringe sectional area.

As a result of the foregoing, the movement of the piston SK of the syringe S can be accurately controlled.

<Setting at a remaining time>

Similarly, when setting at a remaining amount VNE (ml) to be set is performed by referring to Figure 3, the push-finish position LE (cm) is calculated on the basis of the above stored data presented in a table by using the following equation:

$$\text{Push-finish position LE (cm) = LNE (cm) + R (mL/h) × T}$$

$$\text{(h)/A (cm}^2),$$

where LNE (cm) is the alarm position, R (mL/h) is the infusion rate, T (h) is remaining time and A (cm$^2$) is the syringe sectional area. As a result of the foregoing, the movement of the piston SK of the syringe S can be accurately controlled.

The comparison operation of a predicted alarm position for the pusher is performed as described above (Step S9). When the pusher reaches the predicted alarm position, an alarm (a warning) is given. When the pusher reaches the push-finish position LE (cm), the threshold value of occlusion detection is automatically changed by the threshold value level changing means 110 (Step S10). After that, the action indicator 7 blinks (lights up) red, thereby giving an alarm (Step S11) and the liquid infusion (administration) is completed (Step S12).

Incidentally, if injection is not started even when about two minutes pass after the completion of flow rate setting after the setting of a syringe S in the main body 1, the buzzer sounds to make a forgotten start known to the user. In this case, the buzzer stops sounding when the stop/mute switch 28 is pressed. When an infusion pattern (a flow rate) is to be changed during infusion, the infusion is stopped by performing temporary stop of infusion and after that, resetting is performed by use of the setting input means 60 including the setting dial 6. At this time, the action indicator 7 goes off when the stop/mute switch 28 is pressed.

When the infusion of a liquid medicine in a predetermined amount is completed, the stop condition is ascertained and the syringe is removed. For this purpose, the clamp 5 is drawn upward and then held in a condition turned by about 90 degrees. Subsequently, the clutch lever 52 of the slider assembly 50 is pressed and the syringe is removed by opening the right and left hook members. After that, when the syringe pump is not used again, the power switch 15 is cut off by being continuously pressed for not less than about 2 seconds.

This syringe pump can also be applied to a case where product information, such as a syringe maker, a syringe diameter (capacity), medicine type, contained in a bar code and an IC chip applied to the syringe is automatically read by use of reading means (not shown).

Now injection per body weight will be described. First, the unit changeover switch (serving also as a display on/off switch) 10a is continuously pressed for not less than 2 seconds, the above-described infusion setting display part 10 lights up and it becomes possible to perform infusion (gamma infusion) per body weight. Subsequently, the display screen of the infusion setting display part 10 changes when the item changeover switch 10b is pressed, and each time the item changeover switch 10b is pressed, an infusion rate (an injected amount) per body weight, body weight, an amount of medicine and an amount of solution are displayed in a blinking manner. Areas where the display is blinking are items whose settings can be changed. Therefore, by turning the setting dial 6 with a desired item kept displayed, numeric values are changed by changing the numeric values on the screen of the infusion setting display part 10. Furthermore, it is possible to rapidly change each step value by turning the setting dial 6 while pressing the stop/mute switch 28. The screen of this gamma infusion setting display part 10 is constructed in such a manner that a flow rate (an infusion rate) is automatically calculated and displayed according to the set infusion amount per body weight, body weight, amount of medicine and amount of solvent.

For the setting of special functions, the operation of a changeover switch built in the interior of the main body enables the setting of various modes to be changed as required. As these special functions, predetermined amounts in the display part 11 of a flow rate, predetermined amount and integrated amount can be changed between a standard mode and a predetermined amount mode. In the standard mode, each time the display changeover switch 26 is pressed, a flow rate and an integrated amount are displayed in this order and it is impossible to set a predetermined amount.

Furthermore, when a changeover switch to the predetermined mode is made by operating the changeover built in the interior of the main body, the display of the display part 11 appears in the order of flow rate, predetermined amount and integrated amount, and it becomes possible to set a predetermined amount. In this setting of a predetermined amount, the predetermined amount lamp 24 is first caused to light up by pressing the display changeover switch 26 and a predetermined amount is then set by turning the setting dial 6. This setting can be changed in 0.1 ml steps. In a case where a predetermined amount is set and injection is started, the predetermined amount lamp 24 blinks and the buzzer sounds when an integrated amount has reached the predetermined amount.

In a case when the setting of a predetermined amount has been performed in this way, the injection of a liquid medicine is continued at a flow rate (an infusion rate) of 0.1 ml/h when the KOR (keep open rate) function works. When the user wants to perform confirmation, the action indicator 7 lights up by the pressing of the stop/mute switch 28 and the buzzer stops sounding. At this time, the keep vein open function continues. When the stop/mute switch 28 is pressed again, the action indicator 7 goes off, the keep vein open function is canceled and comes to a stop condition, and injection in the predetermined amount mode is finished.

A re-alarm permits a change to the standard mode and the mute mode. Specifically, it is possible to set the standard mode with the action indicator 7 blinking red with the buzzer enabled in which when an alarm condition is not canceled for not less than 2 minutes after the buzzer extinguished, the buzzer is caused to sound again; and the mute mode in which the re-alarm function does not to work even when an alarm condition continues for not less than 2 minutes.

A forgotten start alarm permits a change to the standard mode and the mute mode. Specifically, it is possible to set the standard mode with the action indicator 7 blinking red with the buzzer enabled in which when a stop condition continues for not less than 2 minutes in a state permitting a start, the buzzer sounds thereby to make a forgotten start known to the user; and the mute mode in which the forgotten start alarm function does not work even when a stop condition continues for not less than 2 minutes.

A change of a syringe maker name is made possible by use of the built-in changeover switch. However, it is ensured that usually, the setting of this special function cannot be performed, because a trouble might be caused if this function is carelessly performed.

In changing the setting of the sound volume of the buzzer, the stop/mute switch 28 is continuously pressed and the integrating clearing switch 27 is pressed at the same time. Then, "bEL *2" appears on the display part 11 of flow rate, predetermined amount and integrated amount and the setting mode begins. With the stop/mute switch 28 kept pressed, the integrating clearing switch 27 is released and pressed and the display changes to "bEL : 1," "bEL : 2," and "bEL : 3" according to "large," "medium," and "small" of the volume of the buzzer. Therefore, when a desired sound volume is obtained, the user's fingers are released from all switches, whereby the setting of the volume of the buzzer is performed.

The syringe pump can also be operated by use of a DC power source in a centralized power source box. In this case, it is ascertained that an AC power cable is detached from the main body 1 and a DC cable is then connected to a DC connector 9 on the right side surface of the main body. Subsequently, the AC/DC lamp 16 lights up and power is supplied from the DC power source when the power switch 15 is turned on.

When power is supplied neither from the AC power source nor from the DC power source, power supply is automatically changed over to the built-in battery, the AC/DC lamp 16 goes off and the syringe pump can be continuously used for about 3 hours using the built-in battery. Regardless of the on/off state of the power source, the built-in battery can be charged when the AC power source or the DC power source is connected. During this charging the battery lamp 17 lights up and displays the remaining amount of the battery at three levels. A rough standard for the remaining amount of the battery is such that use for not less than about 160 minutes is possible when all the three lamps are on, the action time is for not less than 80 minutes when two lamps are on, and use for not less than 30 minutes is possible when only one lamp is on. A case where all the three lamps blink and a case where two lamps or only one lamp lights up indicate that the battery has deteriorated.

When the remaining amount of the battery decreases further, the battery alarm lamp 22 blinks and the buzzer sounds. Accordingly, the syringe pump is rapidly connected to the AC or DC power source and used.

For an electrostatic noise margin, to prevent a malfunction by contact discharge at $\pm 8$ kV and aerial discharge at $\pm 15$ kV, this syringe pump is constructed so as to conform to the overseas standards: FCC Prat. 18, CISPR 11, IEC 60601-1-2, IEC 60601-2-24, and VDEDIN57871. The electric shockproof type for TYPE CY (ground leakage current: not more than 0.5 mA) in CLASS I (not less than 3 kV) conforms to the domestic standard JIS T1001 and T1002 and the overseas standard IEC 60601-1.

For chemical resistance, wiping off with alcohol is prohibited by operating instructions. However, in order to give the syringe pump chemical resistance to alcohol and similar substances, the outer layer is given ABS grade V0 and measures are taken by performing under printing, for example, so that a nameplate, printed characters, indications, etc. do not disappear.

The driving thrust (the force which pushes the pusher) is about 15 kg·f maximum and the strength of driving mechanism for the syringe pump is constructed so as to withstand about 20 kg·f. The mechanical (feed) accuracy can be maintained at high accuracies of within $\pm 1\%$ by actual measurement and ensured for each syringe type of various syringe makers.

Concretely, by measuring the moving distance of the slider assembly 50, the mechanical (feed) accuracy was set as follows:

(A) Flow (infusion) rate < 1.0 ml/h
60 minutes (long-term): Not more than $\pm 5\%$
(B) 1.0 ml/h $\leq$ flow rate < 5.0 ml/h
2-minute observation from the observation window:

Not more than $\pm 2\%$
and at the same time
60 minutes (long-term): Not more than $\pm 1\%$

(C) 5.0 ml/h ≤ flow rate
2-minute observation from the observation window:

Not more than ±1%
Discharge rates were measured so that high flow (infusion) rate accuracies of within ±3% can be ensured for each syringe type of various syringe makers.

(A) 1.0 ml/h ≤ flow rate < 5.0 ml/h
2-minute observation from the observation window:

Not more than ±5%

(B) 1.0 ml/h ≤ flow rate 60 minutes (long-term): Not more than ±3%
(C) It was ascertained that not less than 95% is ensured for the 10 minutes after the start of infusion.

The display part 10 of an injected amount per body weight is a liquid crystal provided with a back light (yellow or green). This is a numeral indicator composed of seven segments. This display part can display weight in kg, an amount of medicine in mg, an amount of solution in ml, a dose in μg/kg/min or mg/kg/h, etc.; body weight is indicated in 4 digits by the seven segments + a decimal point by one segment; an amount of medicine is indicated in 4 digits by the seven segments + a decimal point by one segment; an amount of solution is indicated in 4 digits by the seven segments + a decimal point by one segment; and a dose is indicated in 4 digits by the seven segments + a decimal point by one segment. Usually, when the ordinary infusion mode is selected, the back light goes off for the whole screen.

The display part 11 of flow rate, integrated amount and predetermined amount is a readily seen LED display, which performs display in 4 digits by seven segments + a decimal point by one segment, changes over automatically to a flow rate display during the setting of the γ mode. Furthermore, during the setting of the mode for injection per weight body (in μg/kg/min or mg/kg/h, etc.) this display part whose flow rate indication changes sequentially in response to a set value is indicated, changes over automatically to a flow rate display if the display part is left as it is for about 15 seconds when an integrated amount or a predetermined amount.

Alarms are given in the case of Er*, an out-of-control CPU, a switch operation and self-check. These alarms are generated in a readily perceived sound free from unpleasant feeling and conform to overseas standards and a changeover at three levels of sound volume (panel operation) is possible. Set sound volume can be stored and the levels of sound volume can be displayed during setting. The maximum sound volume is not less than 65 dB at a distance of 1 m. Furthermore, the alarm system is of the self-excitation type (which gives an alarm in the case of an out-of-control CPU).

The tone quality of the alarms is 3 frequencies (2 to 4 kHz or so). PWM output from the CPU is possible and the following is ensured: alarm → PWM output, watch dog → hardware oscillation. The procedure for sound volume changeover is as follows. (1) In the case of an alarm state (excepting a voltage drop and a remaining amount), a changeover is impossible and the integrating clearing switch is pushed. (2) Each time the integrating clearing switch is pushed, with the stop switch kept pressed, a change occurs from medium → large → small → medium → ... (the buzzer makes a sound for 1.5 seconds). (3) In synchronization with the buzzer, the following appears in the flow rate display part in 7 segments: medium = "b-2," large = "b-3" and small = "b-1." (4) The result is written in E2PROM.

As described above, in a syringe pump and a liquid infusing method according to the invention, by utilizing the characteristic that a detected occlusion value transmitted to the pusher increases beyond a position at which the infusing of a liquid medicine in the syringe is completed, it becomes easy to accurately detect a position at which the infusing of a liquid medicine in the syringe is completed by combining pusher position detection means and occlusion detection means. That is, when a remaining amount decreases beyond a position at which the infusion of a liquid medicine in the syringe is completed, a detected occlusion value transmitted to the pusher increases and this position at which the infusion of a liquid medicine in the syringe is completed may not sometimes be accurately detected. Therefore, a push-finish position until the remaining amount in the syringe is injected can be accurately found by finding the push-finish position from an arithmetic expression by setting a position in which a remaining amount is small as an alarm position, setting an occlusion detection level at a low level and ensuring that the pusher is moved by a slider from the alarm position.

## Claims

1. A syringe pump (1) for infusing a liquid medicine from a syringe (5) according to a predetermined infusing pattern comprising:

a liquid infusing pattern input means (6, 60) arranged for inputting a liquid feeding pattern,
an occlusion pressure setting means (110) arranged for setting and changing of an occlusion detection level, and
a drive control means (70) arranged to control the driving of a motor (218) on the basis of said input liquid infusing pattern and inject the liquid medicine in the syringe by pushing the pusher (SP) of the syringe;
**characterized by**
a pusher (216a) of the syringe pump arranged to move together with said pusher (SP) of the syringe, and
a potentiometer (233) having a lever (234) arranged to detect a position of said pusher (SP) of the syringe and to detect that the pusher of the syringe reaches a predetermined position when said pusher (216a) of the syringe pump contacts and moves said lever (234),

wherein said occlusion pressure changing means (110) is arranged to change said occlusion detection level automatically when said pusher (SP) of the syringe is detected to reach said predetermined position.

2. The syringe pump in accordance with claim 1, further comprising:

a syringe diameter detection means (50) arranged to detect the diameter of the syringe, and
an alarm position setting means (30B) arranged to set the position of said pusher (SP) of the syringe in which a remaining amount of liquid medicine in the syringe decreases as an alarm position, and

wherein said occlusion pressure setting means (110) is arranged to set said occlusion detection level at a low level, and a push-finish position until said remaining amount is infused by the movement of the pusher (SP) of the syringe from said alarm position by use of said drive control means (70) is calculated from a relational expression of $LE = LNE + VNE/A$, in which VNE is the remaining amount, LNE is said alarm position, LE is said push-finish position and A is a cross-sectional area of the syringe (5) derived from the detected diameter of the syringe (5).

3. The syringe pump in accordance with claim 1, further comprising:

a syringe diameter detection means (50) arranged to detect the diameter of the syringe, and
an alarm position setting means (30B) arranged to set the position of said pusher (SP) in which a remaining amount of liquid medicine in the syringe (5) decreases as an alarm position, and

wherein said occlusion pressure setting means (110) is arranged to set said occlusion detection level at a low level, and that a push-finish position until said remaining amount is infused by the movement of the pusher (SP) of the syringe from said alarm position by use of said drive control means (70) is calculated from a relational expression $LE = LNE + R \times T/A$, in which LNE is said alarm position, LE is said push-finish position, R is an infusion rate, T is remaining time and A is a cross-sectional area of the syringe (5) derived from the detected diameter of the syringe.

**Patentansprüche**

1. Spritzenpumpe (1) zum Infundieren eines flüssigen Medikaments aus einer Spritze (5) gemäß einem vorbestimmten Infusionsmuster, umfassend:

ein Flüssigkeitsinfusionsmustereingabemittel (6, 60), das angeordnet ist zum Eingeben eines Flüssigkeitszufuhrmusters,
ein Verstopfungsdruckeinstellmittel (110), das angeordnet ist zum Einstellen und Ändern eines Verstopfungserfassungsniveaus, und
ein Antriebssteuermittel (70), das angeordnet ist zum Steuern des Antriebs eines Motors (218) auf Basis des eingegebenen Flüssigkeitsinfusionsmusters und zum Einspritzen des flüssigen Medikaments in der Spritze durch Vorschieben des Vordrückers (SP) der Spritze;
**gekennzeichnet, durch**
einen Vordrücker (216a) der Spritzenpumpe, der angeordnet ist zum gemeinsamen Bewegen mit dem Vordrücker (SP) der Spritze, und
ein Potentiometer (233) mit einem Hebel (234), das angeordnet ist zum Erfassen einer Position des Vordrückers (SP) der Spritze und zum Erfassen, dass der Vordrücker der Spritze eine vorbestimmte Position erreicht, wenn der Vordrücker (216a) der Spritzenpumpe den Hebel (234) kontaktiert und bewegt,

wobei das Verstopfungsdruckänderungsmittel (110) angeordnet ist zum automatischen Ändern des Verstopfungserfassungsniveaus, wenn erfasst wird, dass der Vordrücker (SP) der Spritze die vorbestimmte Position erreicht.

2. Spritzenpumpe nach Anspruch 1, die ferner umfasst:

ein Spritzendurchmessererfassungsmittel (50), das angeordnet ist zum Erfassen des Durchmessers der Spritze, und

ein Alarmpositionseinstellmittel (30B), das angeordnet ist zum Einstellen der Position des Vordrückers (SP) der Spritze, bei der eine verbleibende Menge des flüssigen Medikaments in der Spritze abnimmt, als eine Alarmposition, und

wobei das Verstopfungsdruckeinstellmittel (110) zum Einstellen des Verstopfungserfassungsniveaus auf ein niedriges Niveau angeordnet ist und eine Vorschubendposition, bis zu der die verbleibende Menge ausgehend von der Alarmposition durch die Bewegung des Vordrückers (SP) der Spritze unter Verwendung des Antriebssteuermittel (70) infundiert ist, berechnet wird aus dem Vergleichsausdruck LE = LNE + VNE/A, in welchem VNE die verbleibende Menge ist, LNE die Alarmposition ist, LE die Vorschubendposition ist und A ein Querschnittsfläche der Spritze (5) ist, die von dem erfassten Durchmesser der Spritze (5) abgeleitet ist.

3. Spritzenpumpe nach Anspruch 1, die ferner umfasst:

ein Spritzendurchmessererfassungsmittel (50), das angeordnet ist zum Erfassen des Durchmessers der Spritze, und

ein Alarmpositionseinstellmittel (30B), das angeordnet ist zum Einstellen der Position des Vordrückers (SP), bei der eine verbleibende Menge des flüssigen Medikaments in der Spritze (5) abnimmt, als eine Alarmposition, und

wobei das Verstopfungsdruckeinstellmittel (110) zum Einstellen des Verstopfungserfassungsniveaus auf ein niedriges Niveau angeordnet ist und wobei eine Vorschubendposition, bis zu der die verbleibende Menge ausgehend von der Alarmposition durch die Bewegung des Vordrückers (SP) der Spritze unter Verwendung des Antriebssteuermittel (70) infundiert ist, berechnet wird aus dem Vergleichsausdruck LE = LNE + R x T/A, in welchem LNE die Alarmposition ist, LE die Vorschubendposition ist, R eine Infusionsrate ist, T die verbleibende Zeit ist und A ein Querschnittsfläche der Spritze (5) ist, die von dem erfassten Durchmesser der Spritze abgeleitet ist.

**Revendications**

1. Pompe à seringue (1) pour administrer par perfusion un médicament liquide depuis une seringue (5) selon un programme de perfusion prédéterminé comprenant :

un moyen d'entrée de programme de perfusion liquide (6, 60) prévu pour entrer un programme de distribution de liquide,
un moyen de réglage de pression occlusive (110) prévu pour établir et modifier un niveau de détection d'occlusion, et
un moyen de commande d'entraînement (70) adapté pour commander l'entraînement d'un moteur (218) en se basant sur ledit programme de perfusion liquide entré et injecter le médicament liquide dans la seringue en poussant le piston (SP) de la seringue ;

**caractérisée par** :

un piston (216a) de la pompe à seringue adapté pour se déplacer en même temps que ledit piston (SP) de la seringue, et
un potentiomètre (233) comportant un levier (234) adapté pour détecter une position dudit piston (SP) de la seringue et pour détecter que le piston de la seringue atteint une position prédéterminée quand ledit piston (216a) de la pompe à seringue entre en contact avec ledit levier (234) et déplace ce dernier,

dans laquelle ledit moyen de réglage de pression occlusive (110) est adapté pour modifier ledit niveau de détection d'occlusion de manière automatique quand il est détecté que le piston (SP) de la seringue atteint ladite position prédéterminée.

**2.** Pompe à seringue selon la revendication 1, comprenant en outre :

un moyen de détection de diamètre de seringue (50) adapté pour détecter le diamètre de la seringue, et
un moyen de réglage de position d'alarme (30B) adapté pour régler la position du piston (SP) de la seringue dans laquelle la quantité d'un reste de médicament liquide dans la seringue diminue en tant que position d'alarme, et

dans laquelle ledit moyen de réglage de pression occlusive (110) est adapté pour établir ledit niveau de détection d'occlusion à un niveau bas, et une position de fin de poussée jusqu'à ce que ladite quantité restante soit injectée par le déplacement du piston (SP) de la seringue à partir de ladite position d'alarme au moyen dudit moyen de commande d'entraînement (70) est calculée à partir de la relation LE = LNE + VNE/A, dans laquelle VNE est la quantité restante, LNE est ladite position d'alarme, LE est ladite position de fin de poussée et A est une section de la seringue (5) obtenue à partir du diamètre détecté de la seringue (5).

**3.** Pompe à seringue selon la revendication 1, comprenant en outre :

un moyen de détection de diamètre de seringue (50) adapté pour détecter le diamètre de la seringue, et
un moyen de réglage de position d'alarme (30B) adapté pour régler la position dudit piston (SP) dans laquelle la quantité restante de médicament liquide dans la seringue (5) diminue en tant que position d'alarme, et

dans laquelle ledit moyen de réglage de pression occlusive (110) est adapté pour établir ledit niveau de détection d'occlusion à un niveau bas, et une position de fin de poussée jusqu'à ce que ladite quantité restante soit injectée par le déplacement du piston (SP) de la seringue à partir de ladite position d'alarme au moyen dudit moyen de commande d'entraînement (70) est calculée à partir de la relation LE = LNE + R x T/A, dans laquelle LNE est ladite position d'alarme, LE est ladite position de fin de poussée, R est une vitesse d'injection, T est un temps restant et A est une section de la seringue (5) obtenue à partir du diamètre détecté de la seringue.

# F I G. 1

# F I G. 2A

# F I G. 2B

# FIG. 3

EP 1 362 606 B1

# FIG. 4

EP 1 362 606 B1

# F I G.  5

```
                    ┌──────────────┐
                    │    START     │
                    └──────┬───────┘
                           ▼
        ┌──────────────────────────────────────┐
        │             SELF-CHECK               │ ～S1
        └──────────────────┬───────────────────┘
                           ▼
                      ╱─────────╲                    S2
                   ╱──────────────╲              YES
                ╱                    ╲ ─────────────────┐
               ╱  IS MAKER NAME CORRECT ? ╲             │
                ╲                    ╱                  │
                   ╲──────────────╱                     │
                      ╲─────────╱                       │
                           ▼ NO                         │
        ┌──────────────────────────────────────┐       │
        │          INPUT MAKER NAME            │ ～S3   │
        └──────────────────┬───────────────────┘       │
                           ▼◄────────────────────┐     │
        ┌──────────────────────────────────────┐ │     │
        │        DETECT SYRINGE DIAMETER       │ ～S4  │
        └──────────────────┬───────────────────┘ │     │
                           ▼◄──────────────────┐ │     │
        ┌──────────────────────────────────────┐ │  ◄──┘
        │       SET LIQUID-INFUSING PATTERN    │ ～S5  │
        └──────────────────┬───────────────────┘       │
                           ▼                            │
        ┌──────────────────────────────────────┐       │
        │    CALCULATE PREDICTED ALARM POSITION │ ～S6  │
        └──────────────────┬───────────────────┘       │
                           ▼                            │
        ┌──────────────────────────────────────┐       │
        │         START LIQUID-INFUSING        │ ～S7  │
        └──────────────────┬───────────────────┘       │
                           ▼                            │
                      ╱─────────╲                    S8 │
                   ╱──────────────╲              YES    │
                ╱    IS LIQUID-INFUSING  ╲ ─────────────┘
               ╱    PATTERN SET AGAIN ?   ╲
                ╲                    ╱
                   ╲──────────────╱
                      ╲─────────╱
                           ▼ NO
                      ╱─────────╲                    S9
                   ╱──────────────╲              NO
                ╱    IS PUSHER POSITION   ╲ ─────────────┐
               ╱   COMPARISON-OPERATED ?   ╲             │
                ╲                    ╱                    │
                   ╲──────────────╱                      │
                      ╲─────────╱                        │
                           ▼ YES                         │
        ┌──────────────────────────────────────┐        │
        │    CHANGE OCCLUSION PRESSURE LEVEL    │ ～S10  │
        └──────────────────┬───────────────────┘        │
                           ▼◄───────────────────────────┘
           NO         ╱─────────╲                   S11
     ┌──────────── ╱──────────────╲
     │          ╱      ALARM ?       ╲
     │           ╲                  ╱
     │              ╲──────────────╱
     │                 ▼ YES
     │          ┌──────────────┐
     │          │     END      │ ～S12
     │          └──────────────┘
     └──────────────────(back to S7)
```

# FIG. 6

| 40 | 50 | 60 | 100 | 110 |
|---|---|---|---|---|
| MAKER INPUT MEANS | SYRINGE DIAMETER DETECTION MEANS | SETTING INPUT MEANS | STORAGE MEANS | OCCLUSION PRESSURE LEVEL CHANGING MEANS |

90

CPU

| COMPARISON MEANS | PUSHER POSITION DETECTION MEANS | FORETOLD POSITION CALCULATING MEANS | DRIVING MEANS | DISPLAY MEANS | ABNORMAL ACTION DETECTION MEANS |
|---|---|---|---|---|---|
| 20 | 30A | 30B | 70 | 10, 11 | 80 |

EP 1 362 606 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2785114 B
- JP 62044505 A
- JP 1017380 A
- EP 1110569 A